# EUROPÄISCHE PATENTSCHRIFT

(11) **EP 1 716 879 B1**
(45) Veröffentlichungstag und Bekanntmachung des Hinweises auf die Patenterteilung: **05.11.2008**
(21) Anmeldenummer: 05009039.8
(22) Anmeldetag: 25.04.2005
(51) Int. Cl.: A61M 5/142

(54) **Infusionsgerät mit wasserdichter Hülle**
Infusion device with watertight enclosure
Dispositif d'infusion avec boîtier étanche

(43) Veröffentlichungstag der Anmeldung: 02.11.2006
(73) Patentinhaber: F. Hoffmann-La Roche AG, 4070 Basel (CH); Roche Diagnostics GmbH, 68305 Mannheim (DE)
(72) Erfinder: Friedli, Kurt, 3421 Lyssach (CH); Haueter, Ulrich, 3506 Grosshöchstetten (CH)
(74) Vertreter: Wess, Wolfgang

(56) Entgegenhaltungen:
- EP-A- 1 486 218
- WO-A-01/13785
- DE-A1- 10 240 165
- US-A- 4 373 527
- US-A- 5 045 051
- US-A1- 2002 077 598

## Beschreibung

Die Erfindung betrifft Infusionsgeräte für therapeutische Verwendungen, vorzugsweise für die Selbstverabreichung von Medikamenten.

Typische Beispiele solcher Infusionsgeräte sind Insulinpumpen, wie sie in der Diabetestherapie eingesetzt werden. Die meisten Insulinpumpen werden als wasserdichte Geräte angeboten. Die Gewährleistung der Wasserdichtigkeit macht mit zunehmender Einsatzdauer der Geräte, die in Zukunft noch weiter steigen dürfte, Probleme. Wenn ein Infusionsgerät als wasserdicht spezifiziert wird, muss die Wasserdichtigkeit über die gesamte Einsatzdauer des Geräts gewährleistet sein. Technisch keine Probleme bereitet es, sicherzustellen, dass jedes Gerät die Produktion wasserdicht verlässt. Probleme können jedoch mit zunehmender Einsatzdauer der Geräte bereits durch Materialermüdung, beispielsweise durch unerkannte Risse im Gehäuse entstehen. Größere Probleme entstehen dadurch, dass solche Geräte oft 24 Stunden täglich in Betrieb und dabei besonderen Belastungen ausgesetzt sein können. Die Geräte können beispielsweise fallen gelassen werden, also Stoßbelastungen erfahren. Nach großen mechanischen Belastungen, insbesondere Stoßbelastungen, kann nicht mehr mit der erforderlichen Sicherheit gewährleistet werden, dass das betreffende Gerät noch wasserdicht ist. Der Eintritt von Feuchtigkeit kann jedoch zu Fehlfunktionen bis hin zum Ausfall eines Geräts führen. Die dosisgenaue Verabreichung des Medikaments wird dadurch gefährdet.

Aus der US 4,3737527 ist ein implantierbares programmierbares Infusionsgerät gemäß den Oberbegriff des Anspruchs 1 bekannt, in dessen wasserdichtem Gehäuse 2 ein Flüssigkeitsdetektor 35 angeordnet ist, der Leckagen des Medikamentenreservoirs 10 oder des dichten Gehäuses 2 detektiert. Die WO 01/13785 A2 beschreibt eine Methode zum Detektieren von Flüssigkeit in einer Vorrichtung. Als Detektoren kommen Indikatoren zum Einsatz, die einen Kontakt mit der Flüssigkeit durch einen Farbumschlag anzeigen.

Es ist eine Aufgabe der Erfindung, bei Infusionsgeräten für therapeutische Verwendungen Störungen durch Feuchteeintritt frühzeitig entgegenzuwirken.

Die Erfindung geht von Infusionsgeräten für therapeutische Verwendungen aus, wie sie grundsätzlich bekannt sind und daher keiner näheren Beschreibung bedürfen. Solch ein Infusionsgerät kann beispielsweise wie bekannte Insulinpumpen ausgebildet sein, die in der Diabetestherapie für die Selbstverabreichung von Insulin verwendet werden. Solch eine Insulinpumpe stellt auch ein bevorzugtes Beispiel eines Infusionsgeräts nach der Erfindung dar. Die Erfindung ist jedoch nicht auf die Verabreichung von Insulin beschränkt, sondern erstreckt sich auf die Verabreichung von Produkten, die per Infusion verabreichbar sind, generell. In erster Linie, aber nicht ausschließlich, ist die Erfindung auf tragbare Geräte für die Selbstverabreichung gerichtet. Vergleichbare Geräte können jedoch auch im stationären Bereich, beispielsweise unter ständiger ärztlicher Aufsicht in Kliniken, beispielsweise in der Rehabilitation oder sonstiger Nachbehandlung, zum Einsatz kommen, wo sie vom Patienten am Körper getragen werden.

Die Erfindung betrifft ein Infusionsgerät mit den Merkmalen nach Anspruch 1.

Das Infusionsgerät ist mit einer wasserdichten Hülle ausgestattet. Die Hülle kann insbesondere das Gehäuse des Infusionsgeräts bilden, das eine Fördereinrichtung für das zu verabreichende Produkt, die erforderlichen Steuerungs- und ggf. Regelungskomponenten sowie eine Bedieneinrichtung und in den meisten Fällen eine optische Anzeige lagert und wasserdicht umhüllt. Die Hülle kann auch nur einen Teil des Gehäuses bilden, in dem eine oder mehrere wasserempfindliche Komponente(n) aufgenommen ist oder sind. Die wasserdichte Hülle kann jedoch auch nur ein wasserdichtes Behältnis für die Aufbewahrung des Infusionsgeräts bilden, wobei in diesem Fall das Infusionsgerät selbst vorzugsweise ein wasserdichtes Gehäuse aufweist, aber nicht aufweisen muss. Wasserdichte Aufbewahrungsbehältnisse sind für den Einsatz im Wasser gedacht, beispielsweise zum Schwimmen, um einen Feuchteeintritt möglichst sicher zu verhindern. Solch ein zusätzliches Aufbewahrungsbehältnis, falls vorhanden, wird im Sinne der Erfindung als zu dem Infusionsgerät gehörig angesehen.

Das Infusionsgerät ist ferner mit einer Überwachungseinrichtung ausgestattet, mittels der die Überwachung der Wasserdichtigkeit der Hülle überwacht wird. Die Überwachungseinrichtung basiert auf einem Medium und umfasst in diesem Sinne ein in der Hülle befindliches Medium, das durch wenigstens eine physikalische oder chemische Größe charakterisiert wird, die sich in Abhängigkeit von der Dichtheit, nämlich der Wasserdichtigkeit oder vorzugsweise sogar der Gasdichtigkeit, der Hülle ändert. Das Medium unterscheidet sich in wenigstens dieser einen physikalischen oder chemischen Größe von der Umgebungsluft.

Die Überwachungseinrichtung umfasst ferner eine Informationseinrichtung, die bei einer Änderung der besagten Größe automatisch zumindest dann ein Informationssignal ausgibt, wenn das absolut und/oder prozentual gesehene Ausmaß der Änderung der Größe den Verlust der Wasserdichtigkeit der Hülle bedeutet. Den Verlust der Wasserdichtigkeit bedeutet es, wenn befürchtet werden muss, dass Wasser oder Wasserdampf aus der Umgebung des Geräts, soweit dort vorhanden, durch die Hülle eindringt. Es soll der Fall eingeschlossen sein, dass Wasser bereits eingetreten ist und als solches oder eine Folge des Eindringens detektiert wird, vorzugsweise wird jedoch die Gefahr eines Wassereintritts präventiv vor einem tatsächlichen Eindringen detektiert. Per Informationssignal wird in geeigneter Weise auf das tatsächliche Eindringen oder die Gefahr eines Eindringens von Wasser hingewiesen.

Das Medium ist ein Gas, das den von der Hülle umhüllten Innenraum füllt. Gegebenenfalls kann ein wasserfreier Dampf verwendet werden, der im folgenden bei Verwendung des Begriffs "Gas" umfasst sein soll. Die Hülle kann mehrwandig, insbesondere doppelwandig, gebildet sein, und das Gas kann in solch einer Ausbildung der Hülle den zwischen zwei benachbarten Wänden der Hülle umschlossenen, in der Hülle selbst gebildeten Innenraum füllen und sozusagen eine Schicht der Hülle bilden.

Die das Gas charakterisierende physikalische oder chemische Größe ist vorzugsweise der Druck, indem das Gas im Innenraum einen anderen Druck als die die Hülle außen umgebende Luft aufweist. Erfindungsgemäß handelt es sich um eine positive Druckdifferenz, d.h. ein Überdruck im Innenraum gegenüber der Umgebung. Ein Überdruck im Innenraum hat den Vorteil, dass durch einen Druckabfall nicht nur ein Leck erkennbar ist, sondern der Überdruck selbst einem Eintritt von Wasser oder Wasserdampf präventiv entgegenwirkt. Ein nicht beanspruchter Unterdruck im Innenraum ermöglicht zwar auch die Leckdetektion, fördert tendenziell jedoch den Feuchteeintritt. Der Differenzdruck, vorzugsweise der positive Differenzdruck, sollte zwischen 0.1 und 1 bar liegen. Bevorzugt werden Differenzdrücke zwischen 0.3 und 1 bar, noch bevorzugter zwischen 0.4 und 0.8 bar.

In einer ersten Ausführung wird der Differenzdruck permanent aufrecht erhalten. Die Überwachungseinrichtung umfasst eine entsprechende Druckeinrichtung für die Aufrechterhaltung des Differenzdrucks. Die Druckeinrichtung arbeitet vorzugsweise so, dass sie bei Erreichen oder Unterschreiten eines vorgegebenen minimalen Differenzdrucks den Druck im Innenraum wieder auf einen vorgegebenen maximalen Differenzdruck bringt. Die Druckeinrichtung sorgt vorzugsweise automatisch für die Aufrechterhaltung des Differenzdrucks, d.h., sie sorgt automatisch dafür, dass ein vorgegebener minimaler Differenzdruck von beispielsweise 0.1 bar oder leicht darunter nie in Richtung Gleichdruck unterschritten wird.

In einer Variante, in der auf eine permanente Aufrechterhaltung eines minimalen Differenzdrucks nicht geachtet wird, wird der Differenzdruck automatisch in vorgegebenen Zeitabständen erzeugt. Wenn der Differenzdruck aufgebaut ist, wird er mittels eines Druckdetektors ebenso automatisch über ein vorgegebenes Zeitintervall überwacht. Dieser Variante gegenüber wird allerdings die automatische permanente Aufrechterhaltung mit permanenter automatischer Überwachung bevorzugt.

In einer zweiten Ausführung wird die Druckdifferenz durch manuelle Betätigung eines Bedienelements oder eines Betätigungselements erzeugt, nämlich "on demand" und/oder automatisch bei Ausführung von durchzuführenden Bedienvorgängen. So kann beispielsweise das Herausnehmen und/oder das Einsetzen eines Reservoirs oder einer Batterie automatisch eine Pumpe betätigen. Nachdem der Differenzdruck eingestellt ist, wird die Dichtheit überprüft, entweder automatisch bei Erreichen eines vorgegebenen Differenzdrucks oder auf Betätigung eines Bedienelements. Die Druckeinrichtung kann eine mittels eines Betätigungselements manuell zu betätigende Druckeinrichtung sein, insbesondere eine mechanische Pumpe, bei der die Pumpbewegung manuell ausgeführt wird, beispielsweise durch Druck auf eine Pumptaste, die in die Hülle integriert und wasserdicht, vorzugsweise gasdicht, nach außen abgeschlossen ist. Die Druckeinrichtung kann in einer Variante halb manuell und halb automatisch arbeiten, indem durch manuelle Betätigung eines Bedienelements die Druckerzeugung veranlasst wird, beispielsweise mittels einer mechanischen, fremdangetriebenen Pumpe, eines Druckreservoirs oder einer anderen Einrichtung zur Druckerzeugung. In der zweiten Ausführung mit manueller Betätigung der Druckeinrichtung oder manueller Auslösung mittels Bedienelement kann ebenfalls für die permanente Aufrechterhaltung eines Differenzdrucks gesorgt werden, indem der Verwender durch ein Signal auf das Unterschreiten eines minimalen Differenzdrucks hingewiesen wird, was allerdings eine entsprechende Überwachung des Drucks im Innenraum erforderlich macht. Eine manuelle Betätigung oder Auslösung mit der Folge, dass ein Überdruck erzeugt wird, kann insbesondere präventiv dann vorgenommen werden, wenn der Verwender des Geräts weiß, dass er demnächst mit Wasser in Berührung kommen wird, beispielsweise weil er schwimmen oder duschen möchte, aber das Infusionsgerät nicht abnehmen möchte.

In beiden Ausführungen einschließlich den Varianten wird der Druck im Innenraum überwacht. Vorzugsweise wird er ständig oder automatisch in vorgegebenen Zeitabständen überwacht. Dabei wird festgestellt, ob ein Abbau der Druckdifferenz rascher vonstatten geht, als bei intakter Hülle zu erwarten ist. Bei der zweiten Ausführung und der Variante der ersten Ausführung reicht es allerdings völlig aus, wenn auch die Drucküberwachung nur stets im Anschluss an die Druckerzeugung für ein vorgegebenes Zeitintervall vorgenommen wird, wobei die Überwachung vorzugsweise automatisch an die Druckerzeugung gekoppelt ist. Grundsätzlich genügt auch in der ersten Ausführung mit permanent aufrecht erhaltenem Differenzdruck eine periodisch oder aperiodisch durchgeführte Drucküberwachung, die vorzugsweise automatisch durchgeführt wird, solange die Zeitabstände zwischen den einzelnen Phasen der Drucküberwachung so klein sind, dass eine Undichtigkeit rechtzeitig festgestellt wird.

Der Differenzdruck kann auf unterschiedliche Art und Weise permanent aufrecht erhalten oder stets neu erzeugt werden. Insbesondere kann der Differenzdruck elektro-thermisch, elektro-chemisch, mechanisch, chemisch oder mittels eines aufquellenden Materials aufrecht erhalten oder erzeugt werden. Auch die Aufrechterhaltung oder Erzeugung durch eine Kombination von zwei oder mehr Verfahren, insbesondere einer Kombination von zwei oder mehr der vorgenannten Verfahren, ist denkbar.

Ein mechanisches Verfahren kann mittels einer Pumpe ausgeführt oder durch Bereitstellung eines Druckreservoirs realisiert werden. Eine mechanische Pumpe zur Erzeugung eines Unter- oder vorzugsweise Überdrucks im Innenraum, die Luft aus dem Innenraum in die Umgebung oder vorzugsweise aus der Umgebung in den Innenraum pumpt, benötigt einen Luftanschluss, der abgedichtet sein muss, beispielsweise mittels eines entsprechenden Ventils, um die geforderte Dichtheit der Hülle zu gewährleisten. Ein Druckreservoir hingegen kann innerhalb der wasserdichten Hülle angeordnet sein und beeinträchtigt nicht deren Dichtheit, könnte andererseits aber möglicherweise die Einsatzdauer des Infusionsgeräts verkürzen oder eine Wartung mit Austausch des Druckreservoirs gegen ein neues erforderlich machen. Die mechanische Pumpe kann eine von außen an den genannten Luftanschluss anschließbare Pumpe sein.

In bevorzugter Ausführung ist im Falle der Verwendung einer mechanischen Pumpe solch eine Pumpe jedoch im Innenraum der Hülle angeordnet. Soll der Differenzdruck automatisch aufrecht erhalten oder erzeugt werden, so kann im Falle einer im Innenraum angeordneten Pumpe der für die Produktförderung vorhandene Antrieb auch als Antrieb der Pumpe zur Aufrechterhaltung oder Erzeugung des Differenzdrucks verwendet werden. Alternativ kann jedoch auch ein zusätzlicher Antrieb vorgesehen sein. Eine handbetätigte mechanische Pumpe wird, wie bereits erwähnt, vorzugsweise durch Drücken einer Drucktaste betätigt, die von einem in Druckrichtung nachgiebigen Teil der Hülle, beispielsweise einer flexiblen Folie, außen überspannt wird. Die mechanische Pumpe kann beispielsweise wie eine einfache Luftpumpe zum Aufpumpen von Reifen, allerdings verkleinert, oder wie die Pumpen von Spraydosen ausgebildet sein.

Eine elektro-chemische Erzeugung zur Aufrechterhaltung eines Überdrucks im Innenraum der Hülle basiert auf wenigstens einem Element, das eine definierte Menge eines Gases erzeugen kann. Im Innenraum kann eine Mehrzahl solcher Elemente angeordnet sein. Als ein solches Element eignet sich insbesondere das System der Gaszelle. Eine Gaszelle ist ein in einem Stromkreis angeordnetes Element, das bei einem Schließen des Stromkreises Wasserstoffgas oder ein anderes Gas produziert. Die Menge des produzierten Gases ist proportional zu dem durch die Zelle fließenden Strom. Solche Elemente sind beispielsweise von automatischen Schmiersystemen, Stichwort "Fettspender", bekannt.

In weniger bevorzugten Ausführungsformen der Erfindung dient als die das Medium charakterisierende physikalische oder mechanische Größe die Konzentration des Gases. Das Gas muss sich natürlich in seiner Zusammensetzung von Luft unterscheiden, beispielsweise indem es ein reines Gas oder eine von Luft unterscheidbare Gasmischung ist. Handelt es sich bei dem Gas um eine Gasmischung, so kann die Konzentration eines einzigen Bestandteils der Mischung die Größe bilden. Als die charakterisierende Größe kann auch die Konzentration eines oder können die Konzentrationen mehrerer der Bestandteile von Luft dienen, dessen Konzentration oder deren Konzentrationen sich beim Eindringen von Luft erhöhen oder überhaupt erst feststellbar ist oder sind. Sinngemäß gilt das Gleiche bei Verwendung eines reinen Gases. Verluste des Gases oder eines Gasbestandteils können insbesondere auf elektro-thermischem, elektro-chemischem oder chemischem Wege oder auch durch die Anordnung eines das Gas enthaltenden Druckreservoirs im Innenraum ausgeglichen werden. Als solch ein Gas, dessen Konzentration im Innenraum überwacht wird, bietet sich beispielsweise gasförmiger Stickstoff an. Auch in den Ausführungen, in denen ein sich in seiner Zusammensetzung von Luft unterscheidendes Gas, nämlich ein reinsortiges Gas oder ein Gasgemisch, das Medium bildet, ist die Aufrechterhaltung eines permanenten Überdrucks im Innenraum erfindungsgemäß notwendig, um dem Eindringen von Feuchtigkeit präventiv entgegenzuwirken.

Die Überwachungseinrichtung umfasst in bevorzugten Ausführungen ferner einen Detektor für die wenigstens eine charakterisierende physikalische oder chemische Größe, insbesondere in Ausführungen, in denen eine Druckdifferenz bzw. ein Druckgradient oder die Konzentration eines Gases oder Gasbestandteils die Größe bildet. Der Ausgang des Detektors ist mit der Informationseinrichtung verbunden. Der Detektor gibt der Informationseinrichtung ein Detektionssignal auf, und die Informationseinrichtung bildet daraus das Informationssignal. Der Detektor kann insbesondere ein integraler Bestandteil der Hülle sein. In bevorzugten Ausführungen bildet er einen Flächenbereich der Hülle, beispielsweise falls es sich um einen Differenzdruckdetektor handelt. Insbesondere kann eine elastische Membran oder ein Dehnmessstreifen ein Sensorelement solch eines Differenzdruckdetektors bilden. Die Membran muss natürlich wasserdicht sein. Vorzugsweise ist sie luftdicht. Falls das Medium ein Gas ist, das leichter als Luft diffundiert oder einen Bestandteil enthält, der leichter als Luft diffundiert, so sollte die Membran dicht auch gegen das betreffende Gas oder den Gasbestandteil mit dem größten Diffusionskoeffizienten sein.

Falls die Konzentration eines den Innenraum füllenden Gases oder eines Bestandteils solch eines Gases die Größe in Verbindung mit vorzugsweise dem Druck als weiterer Größe bildet, sollte ein sauerstofffreies Gas verwendet werden. Das Eindringen von Luft kann in diesem Fall mittels eines Sauerstoffsensors festgestellt werden. Bevorzugt wird mittels des Sauerstoffsensors nicht nur festgestellt, ob die Konzentration von Sauerstoff im Innenraum von dem Wert Null sich unterscheidet, sondern auch ob ein absoluter Schwellwert einfach nur überschritten oder innerhalb eines vorgegebenen Zeitintervalls überschritten wird.

Weitere Möglichkeiten der Überwachung auf Dichtheit, welche, sofern sie auf einem Unterdruck im Innenraum basieren, nicht beansprucht sind, sind die unmittelbare Detektion von Feuchtigkeit mittels Feuchtedetektor oder die Verwendung eines Mediums, das mit Wasser oder Luft, beispielsweise mit dem in der Luft enthaltenen Sauerstoff, chemisch reagiert, wobei die chemische Reaktion und die Reaktionsprodukte unschädlich für das Infusionsgerät als solches sein sollten. Wird die Undichtigkeit über eine chemische Reaktion detektiert, so könnte ein Detektor beispielsweise von einem Thermofühler gebildet werden. Falls die Undichtigkeit unmittelbar mittels Feuchtedetektor detektiert werden soll, bietet sich die Verwendung eines die Feuchte bindenden Mediums an, was gleichzeitig vorteilhafterweise eine präventive Wirkung mit sich bringt, falls nämlich das die Feuchte bindende Medium und der Feuchtedetektor so aufeinander abgestimmt sind, dass die Undichtigkeit detektiert wird, bevor im Innenraum ein gefährlicher Feuchtegrad erreicht wird. Ein geeignetes Medium für die Bindung von Feuchte ist beispielsweise Silicongel.

Falls ein Medium verwendet wird, das bei Vorhandensein von Feuchte, nämlich Wasser oder Wasserdampf, oder Luft einen Farbumschlag zeigt, kann auf den Einsatz eines zusätzlichen Detektors verzichtet werden und der Farbumschlag unmittelbar selbst das Informationssignal bilden. Das Medium bildet selbst den Detektor.

Auf einem zusätzlichen Detektor mit Signalausgang basierende Systeme werden jedoch bevorzugt. Alternativ zu den bereits genannten Detektoren für Druck einschließlich Verformungsdetektoren, Konzentration, Feuchte und Wärme oder zusätzlich zu solch einem Detektor kann ein Detektor vorgesehen sein, der gleichzeitig das Medium bildet und einen Signalausgang aufweist. So können das Medium und der Detektor von einer Schicht der Hülle oder von in die Hülle eingelassenen Partikeln gebildet werden, deren elektrische Leitfähigkeit oder Kapazität oder Induktivität sich bei Kontakt mit Feuchtigkeit ändert und als die physikalische Größe überwacht wird. Sicherzustellen ist allerdings, wie an anderer Stelle bereits erwähnt, dass solch ein Detektormedium über die gesamte Hülle in ausreichender Dichte vorhanden ist.

Das Informationssignal kann ein optisches Signal, ein akustisches oder ein taktiles Signal oder eine Kombination mehrerer dieser Signalarten sein. Ein optisches Informationssignal kann beispielsweise auf einem Display, das üblicherweise bei Infusionsgeräten vorhanden ist, angezeigt werden oder aber mittels einer zusätzlichen Lichtquelle, beispielsweise mittels einer Leuchtdiode. Es sollte als Blinksignal ausgegeben werden. Zur Erzeugung eines taktilen Signals kann beispielsweise ein Vibrator vorgesehen sein. Bevorzugt wird eine Kombination einer optischen Anzeige auf einem Display mit einem akustischen oder taktilen Signal oder eine Kombination eines akustischen und eines taktilen Signals.

Kombinierte Informationssignale bieten die größte Sicherheit, dass der Verwender die Undichtigkeit wahrnimmt und eindeutig als Ursache identifiziert.

Weitere bevorzugte Merkmale der Erfindung werden in den Unteransprüchen und durch die mittels der Unteransprüche beschriebenen Kombinationen von Ansprüchen offenbart.

Vorstehend wurde mehrfach auf die Vorteile von präventiven Maßnahmen hingewiesen, nämlich die Aufrechterhaltung eines permanenten Überdrucks oder die Anordnung eines Feuchtebinders im Innenraum. Auch eine Kombination beider Maßnahmen ist zur Verminderung des Risikos vorteilhaft. Die Anmelderin behält es sich vor, auf die Prävention eine Anmeldung zu richten, ungeachtet der Frage, ob zusätzlich zur Prävention die Dichtheit überwacht wird oder nicht. Allerdings wird auch in Bezug auf solch eine weitere Anmeldung darauf hingewiesen, dass die Kombination aus Prävention und Überwachung als besonders vorteilhaft angesehen wird.

In einer Weiterentwicklung leistet ein in der Hülle bestehender Überdruck die für die Förderung des zu verabreichenden Produkts erforderliche Arbeit. Der Überdruck kann so geregelt eingestellt werden, dass er die Förderung des Produkts exakt mit der gewünschten Förderrate bewirkt. Durch eine Abstimmung des Überdrucks auf die Fördereigenschaften des oder der mechanischen Elemente der Fördereinrichtung kann eine konstante Förderrate und grundsätzlich auch eine variierende Förderrate eingestellt werden, wobei letzteres allerdings bedingt, dass der Überdruck mittel eines wasser- und vorzugsweise wasserdampfdichten Ablassventils im Bedarfsfall auch rasch abgebaut werden kann. Bevorzugt wird es jedoch, wenn der Überdruck wie beschrieben erzeugt und aufrechterhalten wird und dieser Überdruck so groß ist, dass er für die Produktförderung in jeder für die jeweilige Therapie erforderlichen Förderrate ausreicht. Ein Überdruck aus dem Bereich zwischen 0,5 und 5 bar sollte für diesen Zweck genügen.

Falls als mechanisches Förderglied ein mittels des Überdrucks angetriebener Kolben, vorzugsweise Hubkolben, oder ein sonstiges in einem Reservoir bewegbares Förderglied zum Einsatz gelangt, wird der Überdruck vorteilhafterweise auf wenigstens 2 bar eingestellt. Eine derartige Überdruck-Fördereinrichtung sollte andererseits so ausgebildet sein, dass der für den Antrieb des Förderglieds erforderliche Überdruck so gering als möglich ist und vorzugsweise höchstens 4 bar oder noch bevorzugter höchstens 3 bar beträgt. Wird die Förderung des Produkts mittels eines oder mehrerer Kolben bewirkt, ist es vorteilhaft, wenn die Kolbenfläche, auf die der Überdruck wirkt, groß ist. So kann ein das Reservoir bildendes Behältnis beispielsweise in Richtung des Kolbenvortriebs im Vergleich zu wenigstens einer seiner Abmessungen quer zur Vortriebsrichtung sehr flach bauen, so dass die in Vortriebsrichtung sich erstreckende(n), überall runde Seitenwand oder die mehreren kantenbildenden Seitenwände eines derartigen Behältnisses im Verhältnis zu der Kolbenfläche, auf die der Überdruck wirkt, wesentlich kleiner ist oder sind als bei herkömmlichen Produktbehältnissen, beispielsweise zylindrischen Ampullen, wie sie im allgemeinen beispielsweise in der Diabetestherapie eingesetzt werden. Eine derartige Fördereinrichtung kommt mit einem vergleichsweise geringen Überdruck von unter 2 bar aus. Ein besonders bevorzugtes Behältnis dieser Art wird in der deutschen Patentanmeldung Nr. 10 2004 026 805.3 beschrieben, die hiermit in Bezug genommen wird.

In alternativen Ausführungen bildet ein das Produkt aufnehmendes Behältnis selbst das Förderglied der Fördereinrichtung. In solchen Ausführungen ist das Behältnis deformierbar, d.h. mittels des Überdrucks komprimierbar. Das Produkt wird unter der Einwirkung des Überdrucks und des sich dadurch verringernden Innenvolumens des flexiblen Behältnisses aus dem Behältnis verdrängt und ausgeschüttet. So kann insbesondere ein Beutel das Behältnis bilden. Anstatt das Behältnis in seinem das Produkt umhüllenden Volumen insgesamt flexibel zu gestalten, kann auch ein Behältnis verwendet werden, das durch eine Kombination von wenigstens einer steifen Behältniswand und wenigstens einer damit fest verbundenen nachgiebigen Behälterwand gebildet wird. Bei Verwendung eines deformierbaren Behältnisses als Förderglied genügt für die Förderung vorzugsweise der im Zusammenhang mit der Dichtheitsproblematik genannte Überdruck auch zu Zwecken der Produktförderung.

In Kombination mit einer Überdruck-Förderung kommt in vorteilhafter Weiterbildung eine Dosiereinrichtung zum Einsatz, in die das Produkt mittels des Überdrucks undosiert gefördert wird.

Die Dosiereinrichtung kann ein einfaches Absperrorgan sein, das im Auslass des Reservoirs oder stromabwärts von dem Auslass in einer Kanüle angeordnet ist und von der Steuerung zum Schließen und Öffnen des Auslasses oder des Kanülenquerschnitts angesteuert wird.

Bevorzugt wird es jedoch, wenn die Dosiereinrichtung eine zusätzliche Fördereinrichtung bildet, die das mittels der Überdruck-Förderung zugeführte Produkt in kleine Portionen abmisst und die genau abgemessenen Produktportionen weiter fördert. Die von der Dosiereinrichtung gebildeten Produktportionen werden verabreicht. Die Dosiereinrichtung bildet in solch einer Ausführung eine Dosierkammer mit einem ganz bestimmten Kammervolumen. Die Überdruck-Fördereinrichtung, die mit der Steuerung vorteilhafterweise nicht mehr verbunden sein muss, fördert das Produkt in die Dosierkammer, und die Dosiereinrichtung fördert die durch das Dosierkammervolumen vorgegebene Produktmenge portionsweise aus der Dosierkammer in den sich an die Dosierkammer anschließenden Kanülenabschnitt. Mittels der Größe des Kammervolumens und der Taktfrequenz eines Ausstoßelements der Dosiereinrichtung wird die Förderrate bestimmt. Beispiele für geeignete Dosiereinrichtungen sind Peristaltikpumpen, andere Quetschpumpen und insbesondere auch Kolbenpumpen.

Nachfolgend wird die Erfindung anhand von Ausführungsbeispielen erläutert. An den Ausführungsbeispielen offenbar werdende Merkmale bilden je einzeln und in jeder Kombination die Gegenstände der Ansprüche und auch die vorstehend beschriebenen Merkmale der Erfindung in vorteilhafte Richtungen weiter. Es zeigen:
- Fig. 1: ein Infusionsgerät mit einer wasserdichten Hülle und einer Überwachungseinrichtung nach einem ersten Ausführungsbeispiel;
- Fig. 2: ein Infusionsgerät mit einer wasserdichten Hülle und einer Überwachungseinrichtung nach einem zweiten Ausführungsbeispiel;
- Fig. 3: ein Infusionsgerät mit einer wasserdichten Hülle und einer Überwachungseinrichtung nach einem dritten Ausführungsbeispiel;
- Fig. 4: ein Infusionsgerät mit einer Überdruck-Fördereinrichtung und einer Dosiereinrichtung in einem ersten Schaltzustand;
- Fig. 5: die Dosiereinrichtung der Figur 4 in einem zweiten Schaltzustand;
- Fig. 6: die Dosiereinrichtung der Figuren 4 und 5 in einem dritten Schaltzustand; und
- Fig. 7: ein Infusionsgerät mit einer Überdruck-Fördereinrichtung und einer anderen Dosiereinrichtung.

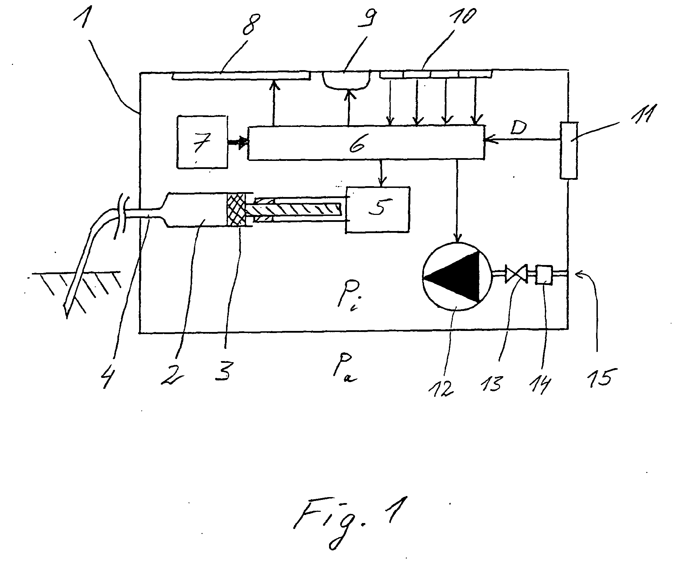

Fig. 1 zeigt ein Infusionsgerät mit einem Gehäuse, das eine wasserdichte Hülle 1 bildet, in der sämtliche Komponenten des Geräts untergebracht sind. Die Hülle 1 umschließt ein Reservoir 2, das mit einem zu verabreichenden Produkt gefüllt ist, ferner eine Fördereinrichtung, eine Überwachungseinrichtung und eine Anzeige- und Bedieneinrichtung. Die Fördereinrichtung umfasst einen in dem Reservoir 2 aufgenommenen Kolben 3, einen Motor 5 und ein Getriebe, über das der Motor 5 für den Antrieb des Kolbens 3 mittels einer Kolbenstange mit dem Kolben 3 gekoppelt ist, eine Steuerung 6 für den Motor 5, die auch eine Leistungselektronik für den Motor 5 umfasst, und eine Energiequelle 7 für die Fördereinrichtung und alle weiteren, mit Energie zu versorgenden Komponenten des Infusionsgeräts. Durch die Hülle 1 ist mit der erforderlichen Abdichtung eine flexible Kanüle 4 geführt, die an einem Ende mit einem Auslass des Reservoirs 2 verbunden ist und an deren anderem Ende eine Infusionskanüle angebracht ist. Die Infusionskanüle ragt für eine subkutane Verabreichung des Produkts unter die Haut eines Patienten. Die Infusionskanüle kann auch unmittelbar aus der Hülle 1 abragen, nur mit der Länge, die in das Gewebe eingeführt wird, um die Hülle 1 direkt auf der Haut anzubringen. Für die Verabreichung wird der Motor 5 von der Steuerung 6 entsprechend einer vorgegebenen Programmierung angesteuert. Der Motor 5 treibt über ein Untersetzungsgetriebe die Kolbenstange und damit zusammen den Kolben 3 in eine Vorschubrichtung auf den Auslass des Reservoirs 2 zu, so dass entsprechend der Kolbenbewegung Produkt aus dem Reservoir 2 verdrängt und subkutan verabreicht wird. Die Steuerung 6 kann insbesondere einen Prozessor und einen oder mehrere zugeordnete Programmspeicher und vorzugsweise auch Datenspeicher umfassen.

Die Anzeige- und Bedieneinrichtung umfasst ein optisches Display 8, einen akustischen Signalgeber 9 und eine Tastatur in Form eines Tastenfelds 10 mit Eingabetasten und Bedientasten. Im Bereich des Displays 8 ist die Hülle 1 durchsichtig. Der akustische Signalgeber 9 und das Tastenfeld 10 sind in die Hülle 1 so integriert, dass zwar ein akustisches Signal abgegeben und die Tasten des Tastenfelds 10 gedrückt werden können, aber andererseits die Wasserdichtigkeit in diesen Bereichen gegeben ist. Das Display 8 kann auch als Touch-screen gebildet sein, so dass auf ein gesondertes Tastenfeld verzichtet werden kann.

Die Hülle 1 wird permanent auf ihre Wasserdichtigkeit überwacht. In dem von der Hülle 1 umhüllten Innenraum herrscht ein Druck Pᵢ, der größer ist als der atmosphärische Druck Pₐ an der äußeren Oberfläche der Hülle 1. Der Druck Pᵢ im Innenraum oder genauer gesagt der Differenzdruck Pᵢ - Pₐ wird ständig überwacht. Für die Überwachung ist das Infusionsgerät mit der Überwachungseinrichtung ausgestattet, deren Komponenten zu einem Teil im Innenraum der Hülle 1 angeordnet und zu einem anderen Teil integraler Bestandteil der Hülle sind. Insoweit wird auch ein Druckgas, im Ausführungsbeispiel Luft, das den Innenraum der Hülle 1 füllt und unter dem Druck Pᵢ steht, zu der Überwachungseinrichtung gerechnet. Der Überdruck Pᵢ ermöglicht nicht nur die Überwachung der Dichtheit durch Überwachung des Drucks Pᵢ oder des Differenzdrucks Pᵢ - Pₐ, sondern ist vorteilhafterweise gleichzeitig auch Prävention gegen das Eindringen von Feuchtigkeit in Form von Wasser oder Wasserdampf aus der äußeren Umgebung.

Die Überwachungseinrichtung umfasst ferner eine Informationseinrichtung und einen Druckdifferenzdetektor 11, dessen Ausgangssignal als Detektionssignal D der Steuerung 6 aufgegeben wird. Die Steuerung 6 hat nämlich nicht nur die Aufgabe, den Motor 5, das Display 8 und den akustischen Signalgeber 9 anzusteuern, um die Fördertätigkeit zu steuern und über das Display 8 und den akustischen Signalgeber 9 die hierfür relevanten Informationssignale einschließlich Alarmsignalen auszugeben, sondern ein Teil der Steuerung 6 bildet gleichzeitig auch eine Komponente der Überwachungseinrichtung. Soweit das Display 8 und der Signalgeber 9 im Rahmen der Überwachung eingesetzt werden, gehören auch sie zur Überwachungseinrichtung für die Dichtigkeit und bilden zusammen mit dem der Überwachung der Dichtigkeit dienenden Teil der Steuerung 6 die Informationseinrichtung der Überwachungseinrichtung. Der Differenzdruckdetektor 11 weist eine luftdichte, elastische Membran auf, die ein Sensorelement des Detektors 11 bildet. Die Membran ist mit der sie umschließenden, festen Struktur der Hülle 1 luftdicht verbunden und elastisch gespannt. Sie wölbt sich aufgrund des Differenzdrucks Pᵢ - Pₐ leicht nach außen. Die Membran kann selbst beispielsweise als Dehnmessstreifen (DMS) ausgebildet sein. Ebenso können Dehnmessstreifen in ein elastisches Membranmaterial eingelassen oder darauf angebracht sein.

Die Steuerung 6, d.h. der zur Überwachungseinrichtung gehörende Teil der Steuerung 6, erkennt anhand eines Vergleichs des Werts des Detektionssignales D mit einem vorgegebenen Vergleichswert, ob die Hülle 1 noch oder nicht mehr dicht ist.

Da eine 100 %ige Luftdichtheit in der Praxis nicht sichergestellt werden kann, umfasst die Überwachungseinrichtung ferner eine Pumpe 12, die durch einen in der Hülle 1 integrierten Luftanschluss 15 mit der Umgebung verbunden ist. In der Verbindungsleitung zwischen dem Luftanschluss 15 und der Pumpe 12 ist ein Verschlussorgan 13 angeordnet, beispielsweise ein einfaches Rückschlagventil, das eine Luftströmung aus dem Innenraum durch die Hülle 1 in die Umgebung verhindert und dadurch die Luftdichtheit der Hülle 1 wahrt. In der Verbindungsleitung ist ferner eine Wassersperre 14 angeordnet, die vorzugsweise die Verbindungsleitung auch gegen ein Eindringen von Wasserdampf sperrt.

Die Steuerung 6 steuert und regelt auch die Pumpe 12 und umfasst eine Leistungselektronik für die Pumpe 12. Die Energiequelle 7 liefert auch die Energie für den Betrieb des Detektors 11 und die Pumpe 12. Dies wird durch die Verbindungsleitung zwischen der Energiequelle 7 und der Steuerung 6 angedeutet. Die Steuerung 6 steuert die Pumpe 12 in Abhängigkeit von dem Wert des Detektionssignals D. In ausreichend kurzen Zeitabständen vergleicht die Steuerung 6 den Wert des Detektionssignals D mit dem vorgegebenen Vergleichswert, der einen vorgegebenen minimalen Überdruck im Innenraum repräsentiert, der nicht unterschritten werden soll. Erreicht der gemessene Überdruck Pᵢ diesen minimalen Wert, so wird die Pumpe 12 angesteuert und fördert Luft aus der Umgebung solange in den Innenraum, bis der Innendruck Pᵢ einen vorgegebenen maximalen Wert erreicht hat. Sobald der Innendruck Pᵢ aufgrund der Fördertätigkeit der Pumpe 12 diesen maximalen Wert erreicht hat, was die Steuerung 6 anhand eines Vergleichs des Werts des Detektionssignals D mit dem maximalen Wert erkennt, schaltet sie die Pumpe 12 ab. Auf diese Weise ist sichergestellt, dass innerhalb der Hülle 1 permanent ein Überdruck Pᵢ herrscht, der allerdings zwischen dem vorgegebenen Maximalwert und dem vorgegebenen Minimalwert schwanken kann.

Um den Verlust der Wasserdichtigkeit der Hülle 1 erkennen zu können, berücksichtigt die Steuerung 6 auch die Zeit, die vergangen ist, wenn ein Druckabfall festgestellt wird. Unterschreitet das Zeitintervall, in dem ein festgestellter Druckverlust stattgefunden hat, ein vorgegebenes Zeitintervall, so bedeutet dies den Verlust der Wasserdichtigkeit. Die Steuerung 6 steuert in solch einem Fall den akustischen Signalgeber 9 in charakteristischer Weise an, so dass der Signalgeber 9 ein den Verlust der Wasserdichtigkeit charakterisierendes akustisches Alarmsignal ausgibt. Ferner steuert die Steuerung 6 das Display 8 in einer charakteristischen Weise an, um den Verlust der Wasserdichtigkeit auch optisch anzuzeigen.

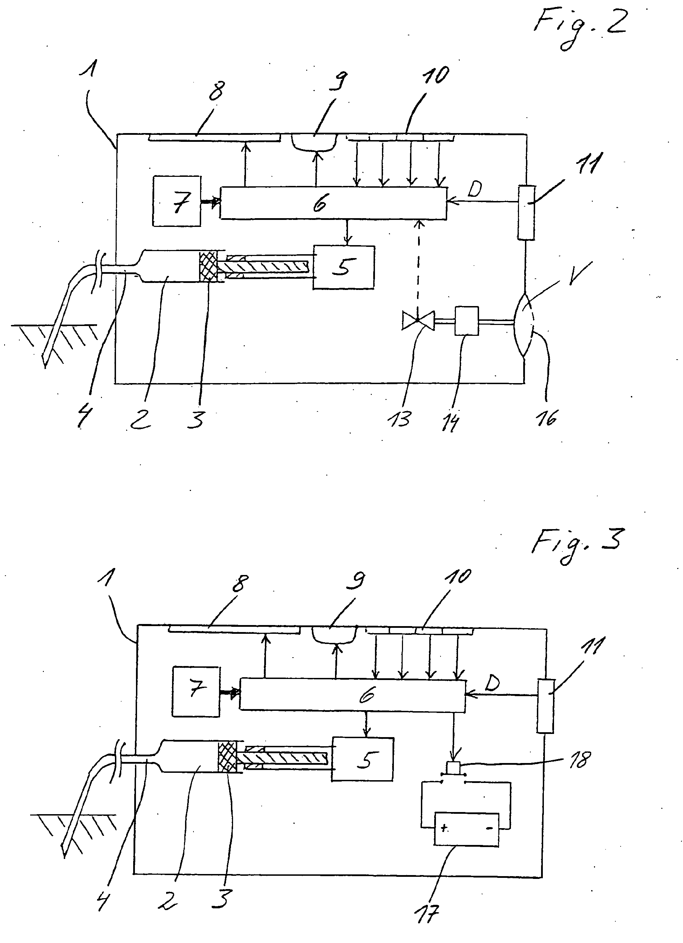

Fig. 2 zeigt ein Infusionsgerät in einem zweiten Ausführungsbeispiel, das sich von dem Infusionsgerät des ersten Ausführungsbeispiels dadurch unterscheidet, dass es anstatt einer motorischen Pumpe eine manuell zu betätigende Pumpe 16 aufweist, die nur im Falle ihrer Betätigung arbeitet. Die Pumpe 16 wird manuell wie eine Drucktaste betätigt. Sie weist eine elastische äußere Membran auf, die in einem mittleren Flächenbereich perforiert ist, sonst aber ein Pumpvolumen V der Pumpe luftdicht nach außen abdichtet. Durch Druck auf die äußere Membran wird deren mittlerer Bereich von der Fingerkuppe des Benutzers abgedichtet und das Volumen V durch die nachgeordnete Wassersperre 14 und das Verschlussorgan 13 in die Hülle 1 gepumpt. Das Rückschlagventil 13 verhindert, dass Gas aus der Hülle in das Pumpvolumen angesaugt werden kann. Die Wasser- und Wasserdampfsperre 14 ist ebenfalls wieder vorgesehen. Die Drucküberwachung kann an die Betätigung der Pumpe 16 gekoppelt sein, so dass sie erst mit der Betätigung oder unmittelbar danach einsetzt und sich dann wieder abschaltet, beispielsweise bei Erreichen und nach einem Anzeigen eines vorgegebenen Differenzdrucks. Diese Betriebsweise dient der Energieeinsparung. Alternativ kann der Druck stattdessen aber auch ständig überwacht werden. Denkbar ist auch zwischen den beiden Modi umzuschalten, beispielsweise am Tastenfeld 10. Angedeutet ist mit strichlierter Linie auch die Alternative, dass das Verschlussorgan 13 der Steuerung 6 mitteilt, dass ein vorgegebener Differenzdruck Pᵢ - Pₐ erreicht ist. So kann das Verschlussorgan 13 beispielsweise als Druckbegrenzungsventil gebildet sein, das bei Erreichen des vorgegebenen Differenzdrucks automatisch den Rückstrom in das Volumen V der Pumpe 16 und somit in die Umgebung freigibt, so dass mit der Pumpe 16 nur der durch das Druckbegrenzungsventil 13 vorgegebene Differenzdruck Pᵢ - Pₐ erzeugt werden kann. Sobald das Verschlussorgan 13 den Rückstrom freigibt, sendet es ein entsprechendes Signal an die Steuerung 6, die über den Signalgeber 9 und/oder das Display 8 anzeigt, dass der vorgegebene Differenzdruck bzw. Solldruck erreicht ist und der Benutzer den Pumpvorgang beenden kann. Obgleich der Innendruck Pᵢ mittels des Detektors 11 periodisch oder vorzugsweise ständig überwacht und bei Unterschreiten eines Minimalwerts eine Anzeige über 8 oder 9 ausgegeben wird, kann auf solch eine Überwachung in einer einfachen Ausführung und auf den Detektor 11 generell auch verzichtet werden. Der erzeugte, positive Differenzdruck Pᵢ - Pₐ wird in solch einer Ausführung rein präventiv gegen ein Eindringen von Feuchtigkeit genutzt.

Das in Fig. 3 dargestellte dritte Ausführungsbeispiel eines Infusionsgeräts unterscheidet sich von dem ersten Ausführungsbeispiel nur durch die Mittel zur Aufrechterhaltung eines permanenten Überdrucks Pᵢ im Innenraum der Hülle 1. Die mechanische Pumpe des ersten Ausführungsbeispiels ist im dritten Ausführungsbeispiel durch eine Gaszelle 17 oder eine Anordnung mehrerer Gaszellen 17 ersetzt, die auf elektro-chemischem Wege ein Gas, beispielsweise H₂-Gas, erzeugt oder erzeugen. Die wenigstens eine Gaszelle 17 ist in einem elektrischen Kreis mit einem Schalter 18 angeordnet. Der Schalter 18 ist mit der Steuerung 6 verbunden. Unterschreitet der mittels des Detektors 11 gemessene Differenzdruck Pᵢ - P_{A} den Minimalwert, schließt die Steuerung 6 mittels des Schalters 18 den elektrischen Kreis, und die wenigstens eine Gaszelle 17 setzt Gas frei. Der Schalter 18 wird gegen eine elastische Rückstellkraft in seiner Schließstellung gehalten, bis der Differenzdruck den Maximalwert erreicht hat. Die Gaszelle 17 oder Gaszellenanordnung ist vorzugsweise in einem auswechselbaren Gehäuseteil, beispielsweise einem Deckel, oder einem lösbar mit dem Gehäuse verbundenen Adapter integriert oder daran innenseitig gehalten.

Von den beschriebenen Unterschieden abgesehen entsprechen das zweite und das dritte Ausführungsbeispiel dem ersten Ausführungsbeispiel.

Der Minimalwert des Differenzdrucks Pᵢ - P_{A} ist vorzugsweise aus dem Bereich von 0.1 bis 0.5 bar gewählt. Der Maximalwert ist vorzugsweise aus dem Bereich von 0.4 bis 1 bar gewählt. Der Detektor 11 kann anstatt ein Differenzdrucksensor auch nur ein Innendrucksensor sein. In diesem Fall wird die Hülle 1 auf einen absoluten Innendruck Pᵢ überwacht, dessen Minimalwert vorzugsweise aus dem Bereich von 1.1 bis 1.5 bar und dessen Maximalwert vorzugsweise aus dem Bereich von 1.4 bis 2 bar und selbstverständlich größer als der Minimalwert gewählt wird.

Der Innenraum der Hülle 1 kann über ein Druckbegrenzungsventil, das von dem Verschlussorgan 13 gebildet sein kann, mit der Umgebung verbunden sein, um einen im Innenraum herrschenden Überdruck zu einem Teil abzubauen. Dies kann unter anderem dann von Vorteil sein, wenn wegen eines zeitweise hohen Umgebungsdrucks, wie er sich beim Tauchen einstellen kann, und wegen einer Einstellung eines Differenzdrucks Pᵢ - P_{A} im Innenraum der Hülle 1 ein hoher Absolutdruck herrscht, der nach dem Auftauchen zu einer unerwünschten Produktausschüttung führen könnte. Mittels des Druckbegrenzungsventils wird der Innendruck in den vorgegebenen Bereich für den Differenzdruck oder den absoluten Druck zurückgeführt.

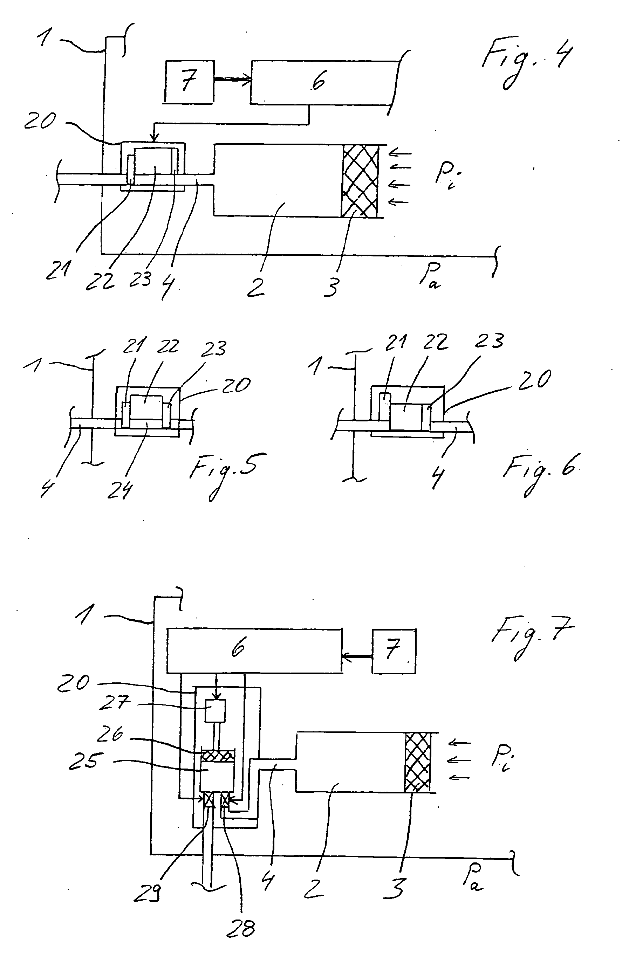

Figur 4 zeigt einen Teil eines Infusionsgeräts, das von den nachstehend beschriebenen Unterschieden abgesehen insbesondere wie die bereits beschriebenen Infusionsgeräte ausgebildet sein kann.

Die Fördereinrichtung umfasst eine einfache Überdruck-Fördereinrichtung und eine Dosiereinrichtung 20. Das im Innenraum der Hülle 1 unter dem Überdruck Pᵢ - Pₐ stehende Fluid bildet das Arbeitsmedium der Überdruck-Fördereinrichtung. Der in dem Reservoir 2 angeordnete Kolben 3 bildet das einzige mechanisch feste Förderglied der Fördereinrichtung. Der Innendruck Pᵢ wirkt unmittelbar auf die Rückseite des Kolbens 3 in Richtung auf den Auslass des Reservoirs 2 zu.

Der Überdruck Pᵢ - Pₐ wird für den Vortrieb des Kolbens 3 auf wenigstens 2 bar eingestellt. Vorteilhaft ist ein Überdruck von etwa 3 bar, gegebenenfalls ein noch größerer Überdruck. Falls der Überdruck geringer sein soll, um beispielsweise die für die Druckerzeugung benötigte Energie und/oder Größe der Druckerzeugungseinrichtung gering halten zu können, wird in alternativen Ausführungen anstatt einer auf der Tätigkeit eines Förderkolbens oder sonstigen mechanischen Förderglieds beruhenden Fördereinrichtung ein unter dem herrschenden Überdruck komprimierbares Reservoir verwendet, d.h. ein Behälter einer ausreichenden Flexibilität. So kann das Reservoir 2 insbesondere durch einen Beutel ersetzt werden, der durch den in der Hülle 1 herrschenden Überdruck so deformiert wird, dass sich sein Volumen verringert und dadurch das Produkt aus dem derart flexiblen Reservoir verdrängt wird. In solchen Ausführungen genügt der an anderen Stellen bereits erwähnte Überdruck von 1 bar oder bevorzugter ein noch geringerer Überdruck auch für die Förderung des Produkts.

Der Überdruck bzw. Differenzdruck Pᵢ - Pₐ ist so groß gewählt, dass er das Produkt mit einer Förderrate aus dem Reservoir 2 fördern würde, die größer als die gewünschte Förderrate ist. Der Differenzdruck beträgt beispielsweise 1 bar, falls ein unter dem herrschenden Überdruck deformierbares Behältnis, beispielsweise ein Beutel, das Reservoir 2 bildet. Falls ein mechanisches Förderglied, beispielsweise ein Kolben 3 oder gegebenenfalls auch ein vom Überdruck angetriebenes Schaufelrad, das Förderglied bildet, kann der Differenzdruck wenigstens 2 bar, vorzugsweise etwa 3 bar, betragen.

Um die Förderrate therapiegemäß einzustellen, beispielsweise eine Basalrate oder einen Sonderbolus, ist dem Reservoir 2 stromabwärts die Dosiereinrichtung 20 nachgeordnet. Die Dosiereinrichtung 20 ist als Peristaltikpumpe gebildet, die innerhalb der Hülle 1 auf einen Abschnitt der flexiblen Kanüle 4 wirkt. Die Dosiereinrichtung 20 umfasst drei Quetschelemente 21, 22 und 23, die in Förderrichtung hintereinander entlang der Kanüle 4 angeordnet sind und auf diese in bekannter Weise quetschend wirken. Jedes der Quetschelemente 21 bis 23 ist zwischen einer ersten Position und einer zweiten Position hin und her bewegbar. In der jeweils ersten Position übt das betreffende Quetschelement 21, 22 oder 23 auf die Kanüle 4 keine Quetschkraft aus. In der jeweils zweiten Position wird die Kanüle 4 dicht abgequetscht. Die Steuerung 6 steuert die Quetschelemente 21-23 einzeln in entweder die erste oder die zweite Position. In dem Schaltzustand der Figur 4 nimmt das stromabwärtigste Schaltelement 21 die zweite Position ein, d. h. es quetscht und verschließt dadurch die Kanüle 4. Das stromaufwärtigste Quetschelement 23 und das mittlere Quetschelement 22 nehmen je ihre erste Position ein. Die Überdruck-Fördereinrichtung fördert somit das Produkt bis gegen das Quetschelement 21. Bis zur Quetschstelle herrscht in der Kanüle 4 der Innendruck Pᵢ, wodurch eine vollkommene Aufweitung der flexiblen Kanüle 4 gewährleistet wird.

Figur 5 zeigt die Dosiereinrichtung 20 in einem zweiten Schaltzustand, der unmittelbar auf den in Figur 4 dargestellten ersten Schaltzustand folgt. Das Quetschelement 21 quetscht und verschließt dadurch immer noch die Kanüle 4. Ferner ist im Vergleich zu dem ersten Schaltzustand auch das Quetschelement 23 in die zweite Position bewegt worden, so dass die Kanüle 4 an einer zweiten Quetschstelle dicht verschlossen ist. Zwischen den beiden Quetschstellen bildet die Kanüle 4 eine Dosierkammer 24 mit einem durch den Kanülenabschnitt und den Abstand der Quetschelemente 21 und 22 vorgegebenen Kammervolumen. Das mittlere Quetschelement 22 nimmt seine erste Position ein.

Figur 6 zeigt die Dosiereinrichtung 20 in einem dritten Schaltzustand, der auf den zweiten Schaltzustand folgt, indem das Quetschelement 21 in seine erste Position und anschließend das mittlere Quetschelement 22 in seine zweite Position, d. h. in seine Quetschposition, bewegt wird. Durch das Öffnen des Strömungsquerschnitts der Kanüle 4 und das Zusammenquetschen der Dosierkammer 24 wird das in der Dosierkammer 24 befindliche Produkt ausgestoßen und eine dem Kammervolumen entsprechende Produktmenge ausgeschüttet. Anschließend steuert die Steuerung 6 die Dosiereinrichtung 20 wieder in den ersten Schaltzustand, indem in einem ersten Schritt das Quetschelement 21 wieder in die zweite Position und anschließend die Quetschelemente 22 und 23 in ihre jeweils erste Position bewegt werden. Durch die Taktfrequenz, mit der die Dosiereinrichtung 20 aus dem ersten Schaltzustand in den dritten Schaltzustand und wieder in den ersten Schaltzustand gebracht wird, bestimmt die Steuerung 6 die Förderrate.

Figur 7 zeigt ein Infusionsgerät mit einer alternativen Dosiereinrichtung 20. Von den nachfolgend beschriebenen Unterschieden abgesehen gelten die Ausführungen zu dem Infusionsgerät der Figuren 4 bis 6.

Die Dosiereinrichtung 20 des Ausführungsbeispiels der Figur 7 ist als Kolbenpumpe gebildet. Die Dosiereinrichtung 20 umfasst eine Dosierkammer 25, einen in der Dosierkammer 25 angeordneten Kolben 26, einen Antrieb 27 und zwei Absperrorgane 28 und 29. Die Steuerung 6 steuert den Antrieb 27 und die Absperrorgane 28 und 29. Der Kolben 26 ist in der Dosierkammer 25 zwischen zwei Endpositionen hin und her bewegbar. Der Antrieb 27 ist über eine Kolbenstange mit dem Kolben 26 gekoppelt und bewirkt dessen Bewegung. Der Antrieb und die Kopplung mit dem Kolben 26 sind so gestaltet, dass der Antrieb 27 den Kolben aus jeder der beiden Endpositionen in die jeweils andere bewegt. Der Antrieb 27 und die Kopplung können alternativ auch als Rückhalte- und Rückholeinrichtung gebildet sein, falls der Kolben 26 durch den im Innenraum der Hülle 1 herrschenden Überdruck Pᵢ - P_{A} in Richtung auf den Auslass der Dosierkammer 25 bewegt wird.

Ein erster Abschnitt der Kanüle 4 verbindet das Reservoir 2 mit der Dosierkammer 25, und ein zweiter Abschnitt der Kanüle 4 schließt sich an einen Auslass der Dosierkammer 25 an und führt das Produkt bis zur Stelle der Verabreichung. Das Absperrorgan 28 verschließt und öffnet entsprechend den Steuersignalen der Steuerung 6 den Abschnitt der Kanüle 4, der das Reservoir 2 mit der Dosierkammer 25 verbindet. Vorzugsweise sitzt das Absperrorgan 28 unmittelbar an einem Einlass in die Dosierkammer 25. Das Absperrorgan 29 sitzt vorzugsweise unmittelbar an dem Auslass der Dosierkammer 25.

In einem ersten Schaltzustand der Dosiereinrichtung 20 sind der Einlass der Dosierkammer 25 geöffnet und der Auslass geschlossen. Durch den Differenzdruck Pᵢ - Pₐ wird das Produkt aus dem Reservoir 2 in die Dosierkammer 25 gefördert und diese dadurch gefüllt. Für die Weiterförderung bringt die Steuerung 6 die Dosiereinrichtung 20 in einen zweiten Schaltzustand, indem der Einlass der Dosierkammer 25 durch das Absperrorgan 28 gesperrt und der Auslass 29 mittels des zuvor im Schließzustand befindlichen Absperrorgans 29 geöffnet wird. In dem zweiten Schaltzustand bewegt der Antrieb 27 den Kolben 26 auf den Auslass zu, oder es wird der Kolben 26 durch den Differenzdruck auf den Auslass zu bewegt, so dass eine dem Kammervolumen der Dosierkammer 25 entsprechende Produktmenge verdrängt und ausgeschüttet wird. Anschließend steuert die Steuerung 6 den Antrieb 27 um, und der Antrieb 27 bewegt den Kolben 26 wieder in die in Figur 7 gezeigte Ausgangsposition. Während dieser Rücksetzbewegung ist das Absperrorgan 28 geöffnet und das Absperrorgan 29 geschlossen, so dass sich die Dosierkammer 25 wieder mit dem Produkt füllt.

## Patentansprüche

1. Infusionsgerät für eine therapeutische Verwendung
mit einer wasserdichten Hülle (1), die ein Gehäuse, Gehäuseteil oder ein Behältnis für die Aufbewahrung des Infusionsgeräts bildet,
und mit einer Überwachungseinrichtung für die Überwachung der Wasserdichtigkeit der Hülle (1),
die Überwachungseinrichtung umfassend:
a) ein in der Hülle (1) befindliches Medium, das durch wenigstens eine physikalische oder chemische Größe gegenüber der Umgebungsluft charakterisiert wird, die sich in Abhängigkeit von der Dichtheit der Hülle (1) ändert,
b) und eine Informationseinrichtung (6, 8, 9), die bei einer Änderung der Größe automatisch zumindest dann ein Informationssignal ausgibt, wenn das Ausmaß der Änderung den Verlust der Wasserdichtigkeit der Hülle (1) bedeutet, wobei
c) das Medium ein Gas ist, das den von der Hülle (1) umhüllten Innenraum füllt, wobei
d) das Gas in dem Innenraum einen Druck (Pᵢ) aufweist, der sich vom Druck (Pₐ) der äußeren Umgebung der Hülle (1) unterscheidet,
**dadurch gekennzeichnet, dass**
es sich bei dem Differenzdruck (Pᵢ-Pₐ) um einen permanenten Überdruck (Pᵢ-Pₐ) handelt.

2. Infusionsgerät nach einem der vorhergehenden Ansprüche, **dadurch gekennzeichnet, dass** die Überwachungseinrichtung einen Detektor (11) umfasst, der eine Änderung eines Werts der physikalischen oder chemischen Größe detektiert und der Informationseinrichtung (6, 8, 9) in Abhängigkeit von dem Wert der Größe ein Detektionssignal (D) aufgibt, aus dem die Informationseinrichtung (6, 8, 9) das Informationssignal bildet.

3. Infusionsgerät nach dem vorhergehenden Anspruch, **dadurch gekennzeichnet, dass** der Detektor (11) einen Teil der Hülle (1) bildet.

4. Infusionsgerät nach einem der vorhergehenden Ansprüche, **dadurch gekennzeichnet, dass** die physikalische oder chemische Größe der Druck ist und die Überwachungseinrichtung einen Druckdetektor (11) für den im Innenraum herrschenden Druck (Pᵢ) oder den Differenzdruck (Pᵢ - Pₐ) zwischen dem Innenraum und der äußeren Umgebung der Hülle (1) umfasst.

5. Infusionsgerät nach einem der vorhergehenden Ansprüche, **dadurch gekennzeichnet, dass** das Gas eine andere Zusammensetzung als Luft aufweist, und dass die physikalische oder chemische Größe die Konzentration des Gases oder wenigstens eines Bestandteils des Gases und/oder eingedrungener Luft ist.

6. Infusionsgerät nach dem vorhergehenden Anspruch, **dadurch gekennzeichnet, dass** die Überwachungseinrichtung einen Detektor für die Konzentration des Gases oder wenigstens eines Bestandteils des Gases und/oder eingedrungener Luft umfasst.

7. Infusionsgerät nach einem der vorhergehenden Ansprüche, **dadurch gekennzeichnet, dass** die Überwachungseinrichtung eine Druckeinrichtung (12; 16; 17) für die Aufrechterhaltung oder Erzeugung eines Differenzdrucks (Pᵢ - Pₐ) umfasst, den der Innenraum zu der äußeren Umgebung der Hülle (1) aufweist.

8. Infusionsgerät nach dem vorhergehenden Anspruch, **dadurch gekennzeichnet, dass** die Überwachungseinrichtung mittels der Druckeinrichtung (12; 17) den Differenzdruck (Pᵢ - Pₐ) automatisch aufrecht erhält oder in vorgegebenen Zeitabständen erzeugt.

9. Infusionsgerät nach dem vorhergehenden Anspruch, **dadurch gekennzeichnet, dass** das Infusionsgerät eine die Druckeinrichtung (12; 17) mit Energie versorgende Energiequelle (7) und eine die Druckeinrichtung (12; 17) in Abhängigkeit von dem Differenzdruck (Pᵢ - Pₐ) steuernde Steuerung (6) umfasst.

10. Infusionsgerät nach einem der drei vorhergehenden Ansprüche, **dadurch gekennzeichnet, dass** die Druckeinrichtung (16) ein Bedien- oder Betätigungselement (10) umfasst und den Differenzdruck (Pᵢ - Pₐ) bei manueller Betätigung des Bedien- oder Betätigungselements (10) erzeugt.

11. Infusionsgerät nach einem der vier vorhergehenden Ansprüche, **dadurch gekennzeichnet, dass** die Druckeinrichtung (16) eine Pumpe (16) umfasst für eine manuell auszuführende Pumpentätigkeit, wobei die Pumpe (16) vorzugsweise wie eine Drucktaste betätigbar ist.

12. Infusionsgerät nach einem der fünf vorhergehenden Ansprüche, **dadurch gekennzeichnet, dass** die Druckeinrichtung (12) eine Pumpe (12) und einen Antrieb (5) für die Pumpe (12) umfasst.

13. Infusionsgerät nach dem vorhergehenden Anspruch, **dadurch gekennzeichnet, dass** der Antrieb (5) auch eine Förderung eines mittels des Infusionsgeräts verabreichbaren Produkts bewirkt.

14. Infusionsgerät nach einem der vorhergehenden Ansprüche, **dadurch gekennzeichnet, dass** die Druckeinrichtung (17) eine elektrochemische Gaszelle (17) umfasst, die ein Gas erzeugt und in dem Innenraum der Hülle (1) freisetzt.

15. Infusionsgerät nach dem vorhergehenden Anspruch, **dadurch gekennzeichnet, dass** eine Fördereinrichtung (3, 5; 3, 20) für die Förderung eines mit dem Infusionsgerät zu verabreichenden Produkts und eine Batterie (7) zur Versorgung eines Antriebs (5; 27) der Fördereinrichtung (3, 5; 3, 20) vorgesehen sind und die Batterie (7) die Gaszelle (17) bildet.

16. Infusionsgerät nach einem der vorhergehenden Ansprüche, **dadurch gekennzeichnet, dass** in der Hülle (1) ein Überdruck (P₁-Pₐ) erzeugt oder aufrechterhalten und mittels des Überdrucks die Förderung eines mit dem Infusionsgerät zu verabreichenden Produkts bewirkt wird.

17. Infusionsgerät nach dem vorhergehenden Anspruch, **dadurch gekennzeichnet, dass** in der Hülle (1) ein Behältnis (2) für das Produkt aufgenommen ist und der Überdruck (P₁-P₂) für die Förderung des Produkts ein in dem Behältnis (2) auf das Produkt wirkendes Förderglied (3) antreibt und/oder das flexibel gebildete Behältnis (2) für die Förderung des Produkts komprimiert.

18. Infusionsgerät nach einem der vorhergehenden Ansprüche, **dadurch gekennzeichnet, dass** die Überwachungseinrichtung das Informationssignal in Abhängigkeit von der Zeit ausgibt, in der der Wert der physikalischen oder chemischen Größe sich um ein vorgegebenes Ausmaß ändert.

19. Infusionsgerät nach einem der vorhergehenden Ansprüche, **dadurch gekennzeichnet, dass** die Überwachungseinrichtung einen in oder an dem Medium angeordneten Feuchtesensor umfasst.

20. Infusionsgerät nach einem der vorhergehenden Ansprüche, **dadurch gekennzeichnet, dass** das Medium ein Gas ist, das den von der Hülle (1) umhüllten Innenraum mit einem Überdruck (Pᵢ - Pₐ) füllt, und das Infusionsgerät ein in der Hülle (1) angeordnetes Reservoir (2) für ein zu verabreichendes Produkt umfasst und der Überdruck (Pᵢ - Pₐ) die Förderung des Produkts bewirkt.

21. Infusionsgerät nach dem vorhergehenden Anspruch, **dadurch gekennzeichnet, dass** das Infusionsgerät eine Dosiereinrichtung (20) umfasst, die eine Dosierkammer (24; 25) mit einem bestimmten Kammervolumen bildet, und dass der Überdruck (Pᵢ - Pₐ) das Produkt in die Dosierkammer (24; 25) und die Dosiereinrichtung (20) das Produkt aus der Dosierkammer (24; 25) fördern.

## Claims

1. An infusion device for a therapeutic application,
comprising a waterproof covering (1) which forms a casing, casing part or a container for storing the infusion device,
and comprising a monitoring means for monitoring the water-impermeability of the covering (1),
the monitoring means comprising:
a) a medium which is situated in the covering (1) and **characterised by** at least one physical or chemical parameter with respect to the ambient air, which changes in accordance with the impermeability of the covering (1);
b) and an information means (6, 8, 9) which, if said parameter changes, automatically outputs an information signal, at least when the extent of the change signifies a loss of water-impermeability in the covering (1), wherein
c) the medium is a gas which fills the inner space enveloped by the covering (1), wherein
d) the gas in the inner space exhibits a pressure (Pᵢ) which differs from the pressure (Pₐ) of the outer surroundings of the covering (1),
**characterised in that**
the differential pressure (Pᵢ - Pₐ) is a permanent pressure burden (Pᵢ - Pₐ).

2. The infusion device according to any one of the preceding claims, **characterised in that** the monitoring means comprises a detector (11) which detects a change in a value of the physical or chemical parameter and feeds a detection signal (D) to the information means (6, 8, 9) in accordance with the value of the parameter, from which the information means (6, 8, 9) forms the information signal.

3. The infusion device according to the preceding claim, **characterised in that** the detector (11) forms a part of the covering (1).

4. The infusion device according to any one of the preceding claims, **characterised in that** the physical or chemical parameter is the pressure, and the monitoring means comprises a pressure detector (11) for the pressure (Pᵢ) prevailing in the inner space or for the differential pressure (Pᵢ - Pₐ) between the inner space and the outer surroundings of the covering (1).

5. The infusion device according to any one of the preceding claims, **characterised in that** the gas exhibits a different composition to that of air, and **in that** the physical or chemical parameter is the concentration of the gas or of at least one constituent of the gas and/or of air which has penetrated in.

6. The infusion device according to the preceding claim, **characterised in that** the monitoring means comprises a detector for the concentration of the gas or of at least one constituent of the gas and/or of air which has penetrated in.

7. The infusion device according to any one of the preceding claims, **characterised in that** the monitoring means comprises a pressure means (12; 16; 17) for maintaining or generating a differential pressure (Pᵢ - Pₐ) which the inner space exhibits relative to the outer surroundings of the covering (1).

8. The infusion device according to the preceding claim, **characterised in that** the monitoring means automatically maintains the differential pressure (Pᵢ - Pₐ), or generates it at predetermined time intervals, by means of the pressure means (12; 17).

9. The infusion device according to the preceding claim, **characterised in that** the infusion device comprises an energy source (7) which supplies the pressure means (12; 17) with energy, and a controller (6) which controls the pressure means (12; 17) in accordance with the differential pressure (Pᵢ - Pₐ).

10. The infusion device according to any one of the preceding three claims, **characterised in that** the pressure means (16) comprises an operating or activating element (10) and generates the differential pressure (Pᵢ - Pₐ) when the operating or activating element (10) is manually activated.

11. The infusion device according to any one of the preceding four claims, **characterised in that** the pressure means (16) comprises a pump (16) for a pump action which is to be performed manually, wherein the pump (16) can preferably be activated like a push key.

12. The infusion device according to any one of the preceding five claims, **characterised in that** the pressure means (12) comprises a pump (12) and a drive (5) for the pump (12).

13. The infusion device according to the preceding claim, **characterised in that** the drive (5) also conveys a product which can be administered by means of the infusion device.

14. The infusion device according to any one of the preceding claims, **characterised in that** the pressure means (17) comprises an electrochemical gas cell (17) which generates a gas and releases it in the inner space of the covering (1).

15. The infusion device according to the preceding claim, **characterised in that** a conveying means (3, 5; 3, 20) for conveying a product to be administered using the infusion device, and a battery (7) for supplying a drive (5; 27) of the conveying means (3, 5; 3, 20) are provided, and the battery (7) forms the gas cell (17).

16. The infusion device according to any one of the preceding claims, **characterised in that** a pressure burden (Pᵢ - Pₐ) is generated or maintained in the covering (1), and a product to be administered using the infusion device is conveyed by means of the pressure burden.

17. The infusion device according to the preceding claim, **characterised in that** a container (2) for the product is accommodated in the covering (1), and the pressure burden (P₁ - P₂) for conveying the product drives a conveying member (3) acting on the product in the container (2) and/or the container (2) is formed to be flexible and is compressed in order to convey the product.

18. The infusion device according to any one of the preceding claims, **characterised in that** the monitoring means outputs the information signal in accordance with the time in which the value of the physical or chemical parameter changes to a predetermined extent.

19. The infusion device according to any one of the preceding claims, **characterised in that** the monitoring means comprises a moisture sensor arranged in or on the medium.

20. The infusion device according to any one of the preceding claims, **characterised in that** the medium is a gas which fills the inner space enveloped by the covering (1) with a pressure burden (Pᵢ - Pₐ), and the infusion device comprises a reservoir (2), arranged in the covering (1), for a product to be administered, and the pressure burden (Pᵢ - Pₐ) conveys the product.

21. The infusion device according to the preceding claim, **characterised in that** the infusion device comprises a dosing means (20) which forms a dosing chamber (24; 25) having a particular chamber volume, and **in that** the pressure burden (Pᵢ - Pₐ) conveys the product into the dosing chamber (24; 25) and the dosing means (20) conveys the product out of the dosing chamber (24; 25).

## Revendications

1. Dispositif de perfusion à usage thérapeutique, comprenant une enveloppe (1) étanche à l'eau qui matérialise un boîtier, une partie de boîtier ou un réceptacle affecté(e) à la conservation dudit dispositif de perfusion, et un système de surveillance conçu pour surveiller l'étanchéité à l'eau de ladite enveloppe (1),
ledit système de surveillance englobant :
a) un fluide renfermé par l'enveloppe (1) et **caractérisé**, vis-à-vis de l'air ambiant, par au moins une grandeur physique ou chimique variant en fonction de l'étanchéité de ladite enveloppe (1),
b) et un système d'information (6, 8, 9) qui, lors d'une variation de ladite grandeur, délivre automatiquement au moins un signal informatif lorsque l'ampleur de ladite variation signifie la perte de l'étanchéité à l'eau de l'enveloppe (1),
c) ledit fluide étant un gaz comblant l'espace interne entouré par l'enveloppe (1),
d) ledit gaz présentant, dans ledit espace interne, une pression (Pᵢ) qui se différencie de la pression (Pₐ) de l'environnement extérieur de l'enveloppe (1),
**caractérisé par le fait que**
la pression différentielle (Pᵢ - Pₐ) se présente comme une surpression permanente (Pᵢ - Pₐ)

2. Dispositif de perfusion selon l'une des revendications précédentes, **caractérisé par le fait que** le système de surveillance comprend un détecteur (11) qui détecte une variation d'une valeur de la grandeur physique ou chimique et qui délivre au système d'information (6, 8, 9), en fonction de la valeur de ladite grandeur, un signal de détection (D) sur la base duquel ledit système d'information (6, 8, 9) forme le signal informatif.

3. Dispositif de perfusion selon la revendication précédente, **caractérisé par le fait que** le détecteur (11) constitue une partie de l'enveloppe (1).

4. Dispositif de perfusion selon l'une des revendications précédentes, **caractérisé par le fait que** la grandeur physique ou chimique est la pression, et le système de surveillance comprend un détecteur de pression (11) assigné à la pression (Pᵢ) régnant dans l'espace interne, ou bien à la pression différentielle (Pᵢ - Pₐ) entre ledit espace interne et l'environnement extérieur de l'enveloppe (1).

5. Dispositif de perfusion selon l'une des revendications précédentes, **caractérisé par le fait que** le gaz présente une composition autre que celle de l'air ; et **par le fait que** la grandeur physique ou chimique est la concentration dudit gaz ou d'au moins un composant dudit gaz et/ou de l'air insinué.

6. Dispositif de perfusion selon la revendication précédente, **caractérisé par le fait que** le système de surveillance comprend un détecteur assigné à la concentration du gaz ou d'au moins un composant dudit gaz et/ou de l'air insinué.

7. Dispositif de perfusion selon l'une des revendications précédentes, **caractérisé par le fait que** le système de surveillance comporte un système de pression (12; 16 ; 17) affecté au maintien ou à la génération d'une pression différentielle (Pᵢ - Pₐ) que l'espace interne présente vis-à-vis de l'environnement extérieur de l'enveloppe (1).

8. Dispositif de perfusion selon la revendication précédente, **caractérisé par le fait que** le système de surveillance entretient la pression différentielle (Pᵢ - Pₐ) ou génère cette dernière à intervalles de temps préétablis, en mode automatique, au moyen du système de pression (12 ; 17).

9. Dispositif de perfusion selon la revendication précédente, **caractérisé par le fait que** ledit dispositif de perfusion comprend une source d'énergie (7) alimentant le système de pression (12 ; 17) en énergie, et une commande (6) pilotant ledit système de pression (12 ; 17) en fonction de la pression différentielle (Pᵢ - Pₐ).

10. Dispositif de perfusion selon l'une des trois revendications précédentes, **caractérisé par le fait que** le système de pression (16) englobe un élément de manoeuvre ou d'actionnement (10), et génère la pression différentielle (Pᵢ - Pₐ) lors d'un actionnement manuel dudit élément de manoeuvre ou d'actionnement (10).

11. Dispositif de perfusion selon l'une des quatre revendications précédentes, **caractérisé par le fait que** le système de pression (16) renferme une pompe (16) destinée à une action de pompage devant être exécutée manuellement, ladite pompe (16) étant de préférence actionnable comme un bouton-poussoir.

12. Dispositif de perfusion selon l'une des cinq revendications précédentes, **caractérisé par le fait que** le système de pression (12) comprend une pompe (12) et un entraînement (5) dédié à ladite pompe (12).

13. Dispositif de perfusion selon la revendication précédente, **caractérisé par le fait que** l'entraînement (5) provoque également un refoulement d'une substance pouvant être administrée au moyen dudit dispositif de perfusion.

14. Dispositif de perfusion selon l'une des revendications précédentes, **caractérisé par le fait que** le système de pression (17) renferme une cellule électrochimique (17) génératrice de gaz, qui génère un gaz et le libère dans l'espace interne de l'enveloppe (1).

15. Dispositif de perfusion selon la revendication précédente, **caractérisé par** la présence d'un système de refoulement (3, 5 ; 3, 20) affecté au refoulement d'une substance devant être administrée par ledit dispositif de perfusion, et par la présence d'une batterie (7) conçue pour l'alimentation d'un entraînement (5 ; 27) dudit système de refoulement (3, 5 ; 3, 20), et ladite batterie (7) matérialise la cellule (17) génératrice de gaz.

16. Dispositif de perfusion selon l'une des revendications précédentes, **caractérisé par le fait qu'**une surpression (P₁ - Pₐ) est engendrée ou entretenue dans l'enveloppe (1), et le refoulement d'une substance à administrer par ledit dispositif de perfusion est provoqué au moyen de ladite surpression.

17. Dispositif de perfusion selon la revendication précédente, **caractérisé par le fait que** l'enveloppe (1) renferme un réceptacle (2) destiné à la substance, et la surpression (P₁ - P₂) affectée au refoulement de ladite substance entraîne un organe refouleur (3) agissant sur ladite substance dans ledit réceptacle (2), et/ou comprime ledit réceptacle (2) de réalisation flexible, en vue du refoulement de ladite substance.

18. Dispositif de perfusion selon l'une des revendications précédentes, **caractérisé par le fait que** le système de surveillance délivre le signal informatif en fonction du temps durant lequel la valeur de la grandeur physique ou chimique varie selon une ampleur préétablie.

19. Dispositif de perfusion selon l'une des revendications précédentes, **caractérisé par le fait que** le système de surveillance englobe un capteur d'humidité placé dans ou sur le fluide.

20. Dispositif de perfusion selon l'une des revendications précédentes, **caractérisé par le fait que** le fluide est un gaz qui comble, avec une surpression (Pᵢ - Pₐ) l'espace interne entouré par l'enveloppe (1), et ledit dispositif de perfusion englobe un réservoir (2) situé dans ladite enveloppe (1) et destiné à une substance devant être administrée, ladite surpression (Pᵢ - Pₐ) provoquant le refoulement de ladite substance.

21. Dispositif de perfusion selon la revendication précédente, **caractérisé par le fait que** ledit dispositif de perfusion comprend un système de dosage (20) matérialisant une chambre de dosage (24 ; 25) dotée d'un volume déterminé ; et **par le fait que** la surpression (Pᵢ - Pₐ) refoule la substance dans ladite chambre de dosage (24 ; 25), et ledit système de dosage (20) refoule ladite substance hors de ladite chambre de dosage (24 ; 25).
